# EUROPEAN PATENT SPECIFICATION

(11) **EP 3 749 296 B1**
(45) Date of publication and mention of the grant of the patent: **10.11.2021**
(21) Application number: 19706408.2
(22) Date of filing: 07.02.2019
(51) Int. Cl.: A61K 31/216, A61P 31/16

(54) **ESTERS OF HYDROXY-BENZOIC ACIDS FOR USE IN THE TREATMENT OF RHINOVIRUS**
ESTER VON HYDROXY-BENZOESÄUREN ZUR VERWENDUNG IN DER BEHANDLUNG VON RHINOVIRUS
ESTERS DE L'ACIDE HYDROXY-BENZOÏQUE POUR L'UTILISATION DANS LE TRAITEMENT DU RHINOVIRUS

(30) Priority: 07.02.2018 GB 201801982
(43) Date of publication of application: 16.12.2020
(73) Proprietor: Enzymatica AB, 223 70 Lund (SE)
(72) Inventor: LINDBERG, Fredrik, 239 40 Falsterbo (SE); STHENGEL, Elisabeth, 25438 Helsingborg (SE)
(74) Representative: Potter Clarkson
(86) International application number: PCT/EP2019/053059
(87) International publication number: WO 2019/154937

(56) References cited:
- WO-A1-98/58627
- MAUGERI ET AL: "New anti-viral drugs for the treatment of the common cold", BIOORGANIC & MEDICINAL CHEMISTRY, PERGAMON, GB, vol. 16, no. 6, 23 December 2007 (2007-12-23), pages 3091-3107, XP022558544, ISSN: 0968-0896, DOI: 10.1016/J.BMC.2007.12.030
- CHOI H J ET AL: "Anti-human rhinovirus activity of gallic acid possessing antioxidant capacity", PHYTOTHERAPY RESEARCH, WILEY, UK, 1 February 2012 (2012-02-01), pages 1292-1296, XP018503081, ISSN: 0951-418X, DOI: 10.1002/PTR.3101

## Description

### Field of Invention

The present invention is defined in the appended claims. It relates to compounds for use in the treatment or prevention of a rhinovirus infection in a mammal. In particular, the invention provides ester derivatives of hydroxybenzoic acid for use in the treatment or prevention of a rhinovirus infection in a mammal.

### Background

The rhinovirus is the most common viral infectious agent in humans and is the predominant cause of the common cold. Rhinovirus infection proliferates in temperatures between 33-35°C (91-95°F), the temperatures found in the nose.

Rhinoviruses have single-stranded positive sense RNA genomes of between 7200 and 8500 nucleotides in length. At the 5' end of the genome is a virus-encoded protein, and like mammalian mRNA, there is a 3' poly-A tail. Structural proteins are encoded in the 5' region of the genome and non-structural at the 3' end. This is the same for all picornaviruses. The viral particles themselves are not enveloped and are icosahedral in structure.

The viral proteins are translated as a single, long polypeptide, which is cleaved into the structural and non-structural viral proteins.

Human rhinoviruses are composed of a capsid, that contains four viral proteins VP1, VP2, VP3 and VP4. VP1, VP2, and VP3 form the major part of the protein capsid. The much smaller VP4 protein has a more extended structure and lies at the interface between the capsid and the RNA genome. There are 60 copies of each of these proteins assembled as an icosahedron. Antibodies are a major defence against infection with the epitopes lying on the exterior regions of VP1-VP3.

Effective treatments for rhinoviral infection remain very limited. There is an anecdotal report that gallic acid (a hydroxyl-substituted benzoic acid) may exhibit anti-human rhinovirus activity (see Choi et al., 2010, Phytother. Res. 24(9): 1292-6). However, this research does not appear to have been continued further.

Against this background, the present invention seeks to provide new and effective compounds for treating and preventing rhinovirus infection.

### Summary of Invention

The first aspect of the invention provides an ester derivative of hydroxybenzoic acid for use in the treatment or prevention of a rhinovirus infection in a mammal, for formula I: 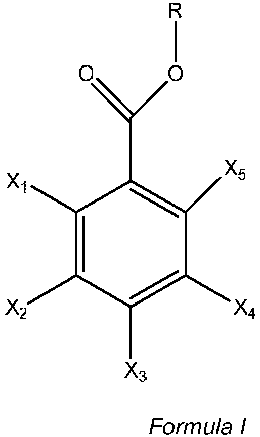 wherein;
R represents a C₁₋₁₀ alkyl group; and
X₁, X₂, X₃, X₄ and X₅ independently represent -H or -OH
and wherein at least one of X₁, X₂, X₃, X₄ and X₅ is -OH.

By "rhinovirus infection" we include that the mammal is infected with or otherwise harbouring a population of rhinovirus, in the genus *Enterovirus* of the *Picornaviridae* family of viruses.

The primary route of entry for human rhinoviruses is the upper respiratory tract (mouth and nose). Rhinovirus A and B bind to ICAM-1 (Inter-Cellular Adhesion Molecule 1) also known as CD54 (Cluster of Differentiation 54) receptors on respiratory epithelial cells while rhinovirus C uses cadherin-related family member 3 (CDHR3) to mediate cellular entry. As the virus replicates and spreads, infected cells release distress signals known as chemokines and cytokines (which in turn activate inflammatory mediators). Cell lysis occurs at the upper respiratory epithelium. Infection occurs rapidly, with the virus adhering to surface receptors within 15 minutes of entering the respiratory tract. High-risk individuals include children and the elderly. Just over 50% of individuals will experience symptoms within 2 days of infection. Only about 5% of cases will have an incubation period of less than 20 hours, and, at the other extreme, it is expected that 5% of cases would have an incubation period of greater than four and a half days.

Human rhinoviruses preferentially grow at 32 °C (89 °F), notably colder than the average human body temperature of 37 °C (98 °F); hence the virus's tendency to infect the upper respiratory tract, where respiratory airflow is in continual contact with the (colder) extrasomatic environment.

Thus, in one embodiment, the rhinovirus infection is the common cold.

Alternatively, the rhinovirus infection may be associated with *(i.e.* present in a subject having) asthma or chronic obstructive pulmonary disorder (COPD).

By "treatment" we include the alleviation, in part or in whole, of the symptoms of rhinovirus infection. Such treatment may include eradication, or slowing of growth, of a rhinovirus population within the body.

By "prevention" we include the reduction in risk of rhinovirus infection developing in a mammal. However, it will be appreciated that such prevention may not be absolute, *i.e.* it may not prevent all such subjects developing a rhinovirus infection. As such, the terms "prevention" and "prophylaxis" may be used interchangeably.

In one embodiment, the mammal is human.

In an alternative embodiment, the mammal is a non-human mammal. Thus, the compounds of the invention may be used in a veterinary setting, for example in the treatment or prevention of a rhinovirus infection in domestic and/or farm animals (including dogs, cats, rabbits, horses, cattle, pigs, sheep and the like). In a preferred embodiment, the mammal is a dog.

In one embodiment, the rhinovirus infection is an infection of the upper and/or lower respiratory tract. Alternatively, or additionally, the rhinovirus infection may be in the gastrointestinal tract.

By "upper respiratory tract" we include the mouth, nose, sinus, middle ear, throat, larynx, and trachea.

By "lower respiratory tract" we include the bronchial tubes (bronchi) and the lungs (bronchi, bronchioles and alveoli), as well as the interstitial tissue of the lungs.

By "gastrointestinal tract" we mean the canal from the mouth to the anus, including the mouth, oesophagus, stomach and intestines.

In one embodiment, the rhinovirus is a human rhinovirus.

Thus, the rhinovirus may be a human Type A rhinovirus, for example selected from the group consisting of human rhinovirus serotypes HRV-A1, HRV-A2, HRV-A7, HRV-A8, HRV-A9, HRV-A10, HRV-A11, HRV-A12, HRV-A13, HRV-A15, HRV-A16, HRV-A18, HRV-A19, HRV-A20, HRV-A21, HRV-A22, HRV-A23, HRV-A24, HRV-A25, HRV-A28, HRV-A29, HRV-A30, HRV-A31, HRV-A32, HRV-A33, HRV-A34, HRV-A36, HRV-A38, HRV-A39, HRV-A40, HRV-A41, HRV-A43, HRV-A44, HRV-A45, HRV-A46, HRV-A47, HRV-A49, HRV-A50, HRV-A51, HRV-A53, HRV-A54, HRV-A55, HRV-A56, HRV-A57, HRV-A58, HRV-A59, HRV-A60, HRV-A61, HRV-A62, HRV-A63, HRV-A64, HRV-A65, HRV-A66, HRV-A67, HRV-A68, HRV-A71, HRV-A73, HRV-A74, HRV-A75, HRV-A76, HRV-A77, HRV-A78, HRV-A80, HRV-A81, HRV-A82, HRV-A85, HRV-A88, HRV-A89, HRV-A90, HRV-A94, HRV-A95, HRV-A96, HRV-A98, HRV-A100, HRV-A101, HRV-A102 and HRV-A103.

Alternatively, the rhinovirus may be a human Type B rhinovirus, for example selected from the group consisting of human rhinovirus serotypes HRV-B3, HRV-B4, HRV-B5, HRV-B6, HRV-B14, HRV-B17, HRV-B26, HRV-B27, HRV-B35, HRV-B37, HRV-B42, HRV-B48, HRV-B52, HRV-B69, HRV-B70, HRV-B72, HRV-B79, HRV-B83, HRV-B84, HRV-B86, HRV-B91, HRV-B92, HRV-B93, HRV-B97 and HRV-B99.

In a further alternative, the rhinovirus is a human Type C rhinovirus, for example selected from the group consisting of human rhinovirus serotypes HRV-C1, HRV-C2, HRV-C3, HRV-C4, HRV-C5, HRV-C6, HRV-C7, HRV-C8, HRV-C9, HRV-C10, HRV-C11, HRV-C12, HRV-C13, HRV-C14, HRV-C15, HRV-C16, HRV-C17, HRV-C18, HRV-C19, HRV-C20, HRV-C21, HRV-C22, HRV-C23, HRV-C24, HRV-C25, HRV-C26, HRV-C27, HRV-C28, HRV-C29, HRV-C30, HRV-C31, HRV-C32, HRV-C33, HRV-C34, HRV-C35, HRV-C36, HRV-C37, HRV-C38,HRV-C39, HRV-C40, HRV-C41, HRV-C42, HRV-C43, HRV-C44, HRV-C45, HRV-C46, HRV-C47, HRV-C48, HRV-C49, HRV-C50 and HRV-C51.

In one preferred embodiment, the rhinovirus is rhinovirus type 16 (RV-16).

The present invention stems from the unexpected finding that benzoic acid esters commonly used as preservatives, in particular lower alkyl esters of hydroxybenzoic acid, are capable of exhibiting an antiviral effect against rhinovirus.

The lower alkyl moiety, R, within the ester functionality is a C₁₋₂₀ alkyl group.

The term "lower alkyl" is intended to include linear or branched, cyclic or acyclic, C₁-C₂₀ alkyl which may be interrupted by oxygen (preferably no more than five oxygen atoms are present in each alkyl chain).

In one embodiment, R represents an unsubstituted C₁-C₂₀ alkyl group.

Thus, R may represent a C₁₋₈ alkyl group, for example a C₁₋₆ alkyl group, a C₁₋₅ alkyl group, a C₁₋₄ alkyl group, a C₁₋₃ alkyl group, a C₁₋₂ alkyl group or a C₁ alkyl group.

For example, R may represent a methyl group, an ethyl group, a propyl group or a butyl group.

Advantageously, R represents -(CH₂)ₙCH₃, wherein 'n' is an integer between 0 and 3. For example, R may represent -(CH₂)₂CH₃.

In addition to the alkyl ester functionality, the benzoic acid derivatives for use in the present invention also comprise at least one hydroxyl moiety attached to the benzene ring therein.

Thus, the compound may be an alkyl ester of a monohydroxybenzoic acid, for example an alkyl ester of 2-hydroxybenzoic acid (salicylic acid), 3-hydroxybenzoic acid or 4-hydroxybenzoic acid. For example, in one preferred embodiment the compound is an alkyl ester of 4-hydroxybenzoic acid.

Alternatively, the compound may be an alkyl ester of a dihydroxybenzoic acid, for example an alkyl ester of 2,3-hydroxybenzoic acid, 2,4-hydroxybenzoic acid, 2,5-hydroxybenzoic acid or 2,6-hydroxybenzoic acid.

In a further alternative embodiment, the compound is an alkyl ester of a trihydroxybenzoic acid, for example an alkyl ester of 3,4,5-trihydroxybenzoic acid (gallic acid) or 2,4,6-trihydroxybenzoic acid (phloro-glucinol carboxylic acid).

In a still alternative embodiment, the compound is an alkyl ester of a tetrahydroxybenzoic acid

In a still alternative embodiment, the compound is an alkyl ester of a pentahydroxybenzoic acid

In a particularly preferred embodiment, the compound is benzoic acid 4-hydroxy propyl ester (propagin; propyl paraben; N-propyl-p-hydroxy-benzoate):

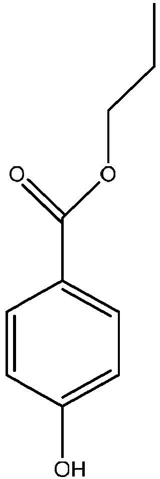

Conveniently, the compounds of the first aspect of the invention are provided in a form suitable for delivery to the mucosa of the mouth and/or pharynx, for example in a mouth spray, nasal spray, lozenge, pastille, chewing gum or liquid.

In one preferred embodiment, the compound is provided as a mouth spray.

It will be appreciated by persons of skill in the art that the compounds of the invention may be used in combination with other active agents, either combined within the same composition/formulation or administered separately.

Thus, in one embodiment, the compound is for use in combination with a polypeptide having protease activity.

By "protease activity" we include any polypeptide which is capable of catalysing proteolysis in vivo, in the mammalian (e.g. human) body. Thus, any type of protease may be utilised in the invention, including but not limited to serine proteases (such as trypsins/chymotrypsins), threonine proteases, cysteine proteases, aspartate proteases, glutamic acid proteases and metalloprotease.

For example, the polypeptide having protease activity may be selected from the group consisting of serine proteases, threonine proteases, cysteine proteases, aspartate proteases, glutamic acid proteases and metalloproteases.

In one embodiment, the polypeptide having protease activity is a serine protease. By "serine protease" we include both naturally occurring and non-naturally occurring catalytic polypeptides capable of cleaving peptide bonds in proteins, in which serine serves as the nucleophilic amino acid at the active site of the polypeptide (as defined in accordance with EC Number 3.4.21). The serine protease may have chymotrypsin-like protease activity *(i.e.* trypsins, chymotrypsins and elastases) or subtilisin-like protease activity.

Thus, in one embodiment the protease is a trypsin or chymotrypsin, or a component of a mixture thereof.

Thus, the polypeptides of the invention may exhibit trypsin activity. By "trypsin activity" we mean that the polypeptide exhibits a peptidase activity of a trypsin enzyme (EC 3,4,21,4) or of a related peptidase (such as chymotrypsin enzymes, EC 3,4,21,1). For example, the protease may be a naturally-occurring trypsin, of either eukaryotic or prokaryotic origin, or a mutated version of such a trypsin.

In one embodiment of the invention, the polypeptide having protease activity is cold-adapted, *i.e.* the polypeptide is psychrophilic. By "cold-adapted" we mean the polypeptide is derived from an organism from a cold environment, and is hence adapted to function at low temperatures. For example, the polypeptide having protease activity may exhibit protease activity for longer periods of time at 15°C than at higher temperatures, such as 25°C or 37°C (see Stefansson et al., 2010, Comparative Biochem. Physiol: Part B - Biochem. & Mol. Biol., 155(2): 186-194(21).

The polypeptides of the invention may be naturally occurring or non-naturally occurring.

In one embodiment, the polypeptide having protease activity comprises or consists of the amino acid sequence of a naturally-occurring protease. For example, the polypeptide having protease activity may consist of the amino acid sequence of a naturally-occurring trypsin, of either eukaryotic or prokaryotic origin.

In one embodiment, the polypeptide is a marine serine protease. The marine serine protease may be obtainable from, for example, cod, pollock, salmon or krill. Other possible sources of marine proteases include catfish, haddock, hoki, hake, redfish, roughies, tilapia, whiting and Chilean seabass. Specifically included are cold-adapted trypsins, such as a trypsin from Atlantic cod *(Gadus morhua),* Atlantic and Pacific salmon *(e.g. Salmo salar* and species of *Oncorhynchus)* and Alaskan Pollock *(Theragra chalcogramma).* For example, the polypeptide having serine protease activity may comprise or consist of the amino acid of SEQ ID NO:1, as listed below.

In a preferred embodiment, the marine serine protease is obtainable from Atlantic cod. Naturally-occurring serine proteases may be purified from a source organism (e.g. Atlantic cod) or may be expressed recombinantly.

Thus, it will be appreciated by persons skilled in the art that such naturally-occurring serine protease polypeptides of the invention must be provided in a form different to that in which they are found in nature. For example, the polypeptide of the invention may consist of the amino acid sequence of a naturally-occurring eukaryotic trypsin but lack the glycosylation moieties present on the protein as it is expressed in nature.

In a preferred embodiment, the marine serine protease is a trypsin, for example trypsin I, trypsin X, trypsin Y or trypsin ZT (for example, see below).

Three major isozymes of trypsin were originally characterised from Atlantic cod, designated Trypsin I, II and III (see Ásgeirsson et al., 1989, Eur. J. Biochem. 180:85-94). For example, trypsin I from Atlantic cod is defined in GenBank Accession No. ACO90397 (see Stefansson etal., 2010, Comp. Biochem. Physiol. B, Biochem. Mol. Biol. 155 (2), 186-194). Subsequently, the trypsins produced by Atlantic cod have been further characterised and a number of distinct isoforms have now been characterised, including trypsin I, trypsin ZT, trypsin X and trypsin Y (see below).

In addition, Atlantic cod expresses two major isozymes of chymotrypsin, designated Chymotrypsin A and B (see Ásgeirsson & Bjarnason, 1991, Comp. Biochem. Physiol. B 998:327-335). For example, see GenBank Accession No. CAA55242.1.

In one embodiment, the polypeptide having protease activity comprises or consists of an amino acid sequence of trypsin I from Atlantic cod (*Gadus morhua*), *i.e.* SEQ ID NO: 1 or SEQ ID NO: 2 
IVGGYECTKHSQAHQVSLNSGYHFCGGSLVSKDWVVSAAHCYKSRIEVRLGEHHIRVNEG
TEQYISSSSVIRHPNYSSYNINNDIMLIKLSKPATLNQYVQPVALPTECAADGTMCTVSG
WGNTMSSVADGDKLQCLSLPILSHADCANSYPGMITQSMFCAGYLEGGKDSCQGDSGGPV
VCNGVLQGVVSWGYGCAERDHPGVYAKVCVLSGWVRDTMANY
[SEQ ID NO: 1]
 
IVGGYECTKHSQAHQVSLNSGYHFCGGSLVSKDWVVSAAHCYKSVLRVRLGEHHIRVNEG
TEQYISSSSVIRHPNYSSYNINNDIMLIKLTKPATLNQYVHAVALPTECAADATMCTVSG
WGNTMSSVADGDKLQCLSLPILSHADCANSYPGMITQSMFCAGYLEGGKDSCQGDSGGPV
VCNGVLQGVVSWGYGCAERDHPGVYAKVCVLSGWVRDTMANY
 [SEQ ID NO: 2] or a fragment, variant, derivative or fusion thereof (or a fusion of said fragment, variant or derivative) of SEQ ID NO: 1 or 2, which retains the trypsin activity of said amino acid sequences.

Further details of trypsin I can be found in (see Guðmundsdóttir et al., 1993, Eur J Biochem. 217(3):1091-7 and Stefansson et al., 2010, Comp. Biochem. Physiol. B, Biochem. Mol. Biol. 155 (2), 186-194).

Alternatively, the polypeptide having protease activity may comprise or consist of an amino acid sequence of a trypsin ZT isoform from Atlantic cod (*Gadus morhua*), *e.g.* SEQ ID NOs: 3 to 7 (see WO 2017/017012 to Enzymatica AB).

SEQ ID NO: 3 is the consensus sequence of the ZT- isoforms, ZT-1 to ZT-4, presented below. 
IX1GGX2X3CEPX4SRPFMASLNYGYHFCGGVLINDQWVLSVAHCWYNPYYMQVMLGEHDL
RVFEGTEQLVKTNTIFWHEX5YDYQTLDYDMMMIKLYHPVEVTQSVAPISLPTGPPDGGM
LCSVSGWGNMAMGEEVNLPTRLQCLDVPIVEX6VX7CX8AX9YPGMISPRMX10CX11GX12M
DGGRDX13CNGDSGSPLVCEGVLTGLVSWGX14GCAX15PNX16PGVYVKVYEX17LSWIQTT
LDANP
[SEQ ID NO: 3]
 wherein
X₁ is selected from I and V;
X₂ is selected from Q and H;
X₃ is selected from D and E;
X₄ is selected from R and N;
X₅ is L;
X₆ is selected from T and P;
X₇ is selected from D and A;
X₈ is selected from E and Q;
X₉ is selected from A and S;
X₁₀ is selected from V and M;
X₁₁ is selected from A and V;
X₁₂ is selected from Y and F;
X₁₃ is selected from A and V;
X₁₄ is selected from Q and R;
X₁₅ is selected from L and E;
X₁₆ is selected from Y and S; and
X₁₇ is selected from Y and F.
Atlantic cod trypsin ZT-1 isoform: 
IVGGHECEPNSRPFMASLNYGYHFCGGVLINDQWVLSVAHCWYNPYYMQVMLGEHDLRVF
EGTEQLVKTNTIFWHELYDYQTLDYDMMMIKLYHPVEVTQSVAPISLPTGPPDGGMLCSV
SGWGNMAMGEEVNLPTRLQCLDVPIVEPVACQASYPGMISPRMMCVGFMDGGRDVCNGDS
GSPLVCEGVLTGLVSWGRGCAEPNSPGVYVKVYEFLSWIQTTLDANP
[SEQ ID NO: 4]
Atlantic cod trypsin ZT-2 isoform: 
IVGGHECEPNSRPFMASLNYGYHFCGGVLINDQWVLSVAHCWYNPYYMQVMLGEHDLRVF
EGTEQLVKTNTIFWHELYDYQTLDYDMMMIKLYHPVEVTQSVAPISLPTGPPDGGMLCSV
SGWGNMAMGEEVNLPTRLQCLDVPIVETVDCEAAYPGMISPRMVCAGYMDGGRDACNGDS
GSPLVCEGVLTGLVSWGQGCALPNYPGVYVKVYEYLSWIQTTLDANP
[SEQ ID NO: 5]
Atlantic cod trypsin ZT-3 isoform: 
IIGGQDCEPRSRPFMASLNYGYHFCGGVLINDQWVLSVAHCWYNPYYMQVMLGEHDLRVF
EGTEQLVKTNTIFWHELYDYQTLDYDMMMIKLYHPVEVTQSVAPISLPTGPPDGGMLCSV
SGWGNMAMGEEVNLPTRLQCLDVPIVEPVACQASYPGMISPRMMCVGFMDGGRDVCNGDS
GSPLVCEGVLTGLVSWGRGCAEPNSPGVYVKVYEFLSWIQTTLDANP
[SEQ ID NO: 6]
Atlantic cod trypsin ZT-4 isoform:

IIGGQDCEPRSRPFMASLNYGYHFCGGVLINDQWVLSVAHCWYNPYYMQVMLGEHDLRVF
EGTEQLVKTNTIFWHELYDYQTLDYDMMMIKLYHPVEVTQSVAPISLPTGPPDGGMLCSV

SGWGNMAMGEEVNLPTRLQCLDVPIVETVDCEAAYPGMISPRMVCAGYMDGGRDACNGDS
GSPLVCEGVLTGLVSWGQGCALPNYPGVYVKVYEYLSWIQTTLDANP
[SEQ ID NO: 7]

It will be appreciated by persons skilled in the art that the polypeptide may be present as a mixture of one or more of the above trypsin ZT isoforms, optionally in combination with trypsins I, X and/or Y.

Alternatively, the polypeptide having protease activity may comprise or consist of an amino acid sequence of trypsin X from Atlantic cod, e.g. SEQ ID NOs: 8 to 11 (see Stefansson et al., 2017, Biochim Biophys Acta. 1865(1):11-19).
Atlantic cod trypsin X: 
IVGGYECTRHSQAHQVSLNSGYHFCGGSLVSKDWVVSAAHCYKSVLRVRLGEHHIRVNEG
TEQFISSSSVIRHPNYSSYNIDNDIMLIKLTEPATLNQYVHAVALPTECAADATMCTVSG
WGNTMSSVDDGDKLQCLNLPILSHADCANSYPGMITQSMFCAGYLEGGKDSCQGDSGGPV
VCNGVLQGVVSWGYGCAERDNPGVYAKVCVLSGWVRDTMASY
[SEQ ID NO: 8]
Atlantic cod trypsin X-1: 
IVGGYECTRHSQAHQVSLNSGYHFCGGSLVSKDWVVSAAHCYKSRIEVRLGEHHIRVNEG
TEQFISSSSVIRHPNYSSYNIDNDIMLIKLSEPATLNQYVQPVALPTECAADGTMCTVSG
WGNTMSSVDDGDKLQCLNLPILSHADCANSYPGMITQSMFCAGYLEGGKDSCQGDSGGPV
VCNGVLQGVVSWGYGCAERDNPGVYAKVCVLSGWVRDTMASY
[SEQ ID NO: 9]
Atlantic cod trypsin X-2: 
IVGGYECTRHSQAHQVSLNSGYHFCGGSLVSKDWVVSAAHCYKSRIEVRLGEHHIRVNEG
TEQFISSSSVIRHPNYSSYNIDNDIMLIKLSKPATLNQYVQTVALPTECAADGTMCTVSG
WGNTMSSVDDGDKLQCLNLPILSHADCSNSYPGMITQSMFCAGYLEGGKDSCQGDSGGPV
VCNGVLQGVVSWGYGCAERDNPGVYAKVCVLSGWVRDTMASY
[SEQ ID NO: 10]
Atlantic cod trypsin X-3:

IVGGYECTRHSQAHQVSLNSGYHFCGGSLVSKDWVVSAAHCYKSRIEVRLGEHHIRVNEG
TEQFISSSSVIRHPNYSSYNIDNDIMLIKLSEPATLNQYVQTVALPTECAADGTMCTVSG
WGNTMSSVDDGDKLQCLNLPILSHADCSNSYPGMITQSMFCAGYLEGGKDSCQGDSGGPV
VCNGVLQGVVSWGYGCAERDNPGVYAKVCVLSGWVRDTMASY
[SEQ ID NO: 11]

Alternatively, the polypeptide having protease activity may comprise or consist of an amino acid sequence of trypsin Y from Atlantic cod, e.g. SEQ ID NO: 12 (see Pálsdóttir & Gudmundsdóttir, 2008, Food Chem. 111(2):408-14).

Atlantic cod trypsin Y:

IIGGQDCEPRSRPFMASLNYGYHFCGGVLINDQWVLSVAHCWYNPYYMQVMLGEHDLRVF
EGTEQLVKTNTIFWHEQYDYQTLDYDMMMIKLYHPVEVTQSVAPISLPTGPPDGGMLCSV
SGWGNMAMGEEVNLPTRLQCLDVPIVETVDCEAAYPGMISPRMVCAGYMDGGRDACNGDS
GSPLVCEGVLTGLVSWGQGCALPNYPGVYVKVYEYLSWIQTTLDANP
[SEQ ID NO: 12]

Thus, in exemplary embodiments, the polypeptide having protease activity comprises or consists of an amino acid sequence according to any one of SEQ ID NOs: 1 to 12. Such a polypeptide may be purified from Atlantic cod, for example as described in Ásgeirsson et al., 1989, Eur. J. Biochem. 180:85-94).

Suitable exemplary polypeptides of the invention, and methods for their production, are also described in European Patent No. 1 202 743 B (7).

Like many proteases, trypsin I from Atlantic cod is produced as an inactive precursor, or zymogen, comprising a propeptide (or "activation") sequence that is cleaved off to generate the mature, active trypsin. The initial expression product for trypsin also comprises a signal sequence, which is removed following expression.

A zymogen sequence for trypsin I from Atlantic cod, including the signal sequence, is shown below as SEQ ID NO:13 (and corresponds to Uniprot database accession no. P16049-1): 
10 20 30 40 50
MKSLIFVLLL GAVFAEEDKI VGGYECTKHS QAHQVSLNSG YHFCGGSLVS
60 70 80 90 100
KDWVVSAAHC YKSVLRVRLG EHHIRVNEGT EQYISSSSVI RHPNYSSYNI
110 120 130 140 150
NNDIMLIKLT KPATLNQYVH AVALPTECAA DATMCTVSGW GNTMSSVADG
160 170 180 190 200
DKLQCLSLPI LSHADCANSY PGMITQSMFC AGYLEGGKDS CQGDSGGPVV
210 220 230 240
CNGVLQGVVS WGYGCAERDH PGVYAKVCVL SGWVRDTMAN Y
[SEQ ID NO: 13]
 wherein:
Signal peptide = amino acids 1 to 13 (underlined)
Propeptide = amino acids 14 to 19 (bold italics)
Mature trypsin = amino acids 20 to 241

The zymogen sequence for the variant trypsin I from Atlantic cod corresponding to SEQ ID NO: 2, including the signal sequence, is shown below as SEQ ID NO: 14 (and corresponds to Uniprot database accession no. P16049-1): 
10 20 30 40 50
MKSLIFVLLL GAVFAEEDKI VGGYECTKHS QAHQVSLNSG YHFCGGSLVS
60 70 80 90 100
KDWVVSAAHC YKSVLRVRLG EHHIRVNEGT EQYISSSSVI RHPNYSSYNI
110 120 130 140 150
NNDIMLIKLT KPATLNQYVH AVALPTECAA DATMCTVSGW GNTMSSVADG
160 170 180 190 200
DKLQCLSLPI LSHADCANSY PGMITQSMFC AGYLEGGKDS CQGDSGGPVV
210 220 230 240
CNGVLQGVVS WGYGCAERDH PGVYAKVCVL SGWVRDTMAN Y
[SEQ ID NO: 14]
 wherein:
Signal peptide = amino acids 1 to 13 (underlined)
Propeptide = amino acids 14 to 19 (bold italics)
Mature trypsin = amino acids 20 to 241

The zymogen sequence for the variant trypsin X corresponding to SEQ ID NO: 8, including the signal sequence, is shown below as SED ID NO: 15 (and corresponds to Genbank Accession No. Q91041.2). 
MKSLIFVLLLGAVFAEEDKIVGGYECTRHSQAHQVSLNSGYHFCGGSLVSKDWVVSAAHC
YKSVLRVRLGEHHIRVNEGTEQFISSSSVIRHPNYSSYNIDNDIMLIKLTEPATLNQYVH
AVALPTECAADATMCTVSGWGNTMSSVDDGDKLQCLNLPILSHADCANSYPGMITQSMFC
AGYLEGGKDSCQGDSGGPVVCNGVLQGVVSWGYGCAERDNPGVYAKVCVLSGWVRDTMAS
Y
[SEQ ID NO: 15]
 (wherein the signal sequence and propeptide are underlined and in bold italics, respectively).

The zymogen sequence for the variant trypsin X-1 corresponding to SEQ ID NO: 9, including the signal sequence, is shown below as SED ID NO: 16 (and corresponds to Genbank Accession No. AOX15769.1) 
MKSLIFVLLLGAVFAEEDKIVGGYECTRHSQAHQVSLNSGYHFCGGSLVSKDWVVSAAHC
YKSRIEVRLGEHHIRVNEGTEQFISSSSVIRHPNYSSYNIDNDIMLIKLSEPATLNQYVQ
PVALPTECAADGTMCTVSGWGNTMSSVDDGDKLQCLNLPILSHADCANSYPGMITQSMFC
AGYLEGGKDSCQGDSGGPVVCNGVLQGVVSWGYGCAERDNPGVYAKVCVLSGWVRDTMAS
Y
[SEQ ID NO: 16]
 (wherein the signal sequence and propeptide are underlined and in bold italics, respectively).

The zymogen sequence for the variant trypsin X-2 corresponding to SEQ ID NO: 10, including the signal sequence, is shown below as SED ID NO: 17 (and corresponds to Genbank Accession No. AOX15770.1) 
MKSLIFVLLLGAVFAEEDKIVGGYECTRHSQAHQVSLNSGYHFCGGSLVSKDWVVSAAHC
YKSRIEVRLGEHHIRVNEGTEQFISSSSVIRHPNYSSYNIDNDIMLIKLSKPATLNQYVQ
TVALPTECAADGTMCTVSGWGNTMSSVDDGDKLQCLNLPILSHADCSNSYPGMITQSMFC
AGYLEGGKDSCQGDSGGPVVCNGVLQGVVSWGYGCAERDNPGVYAKVCVLSGWVRDTMAS
Y
[SEQ ID NO: 17]
 (wherein the signal sequence and propeptide are underlined and in bold italics, respectively).

The zymogen sequence for the variant trypsin X-3 corresponding to SEQ ID NO: 11, including the signal sequence, is shown below as SED ID NO: 18 (and corresponds to Genbank Accession No. AOX15771.1) 
MKSLIFVLLLGAVFAEEDKIVGGYECTRHSQAHQVSLNSGYHFCGGSLVSKDWVVSAAHC
YKSRIEVRLGEHHIRVNEGTEQFISSSSVIRHPNYSSYNIDNDIMLIKLSEPATLNQYVQ
TVALPTECAADGTMCTVSGWGNTMSSVDDGDKLQCLNLPILSHADCSNSYPGMITQSMFC
AGYLEGGKDSCQGDSGGPVVCNGVLQGVVSWGYGCAERDNPGVYAKVCVLSGWVRDTMAS
Y
[SEQ ID NO: 18]
 (wherein the signal sequence and propeptide are underlined and in bold italics, respectively).

The zymogen sequence for the variant trypsin Y corresponding to SEQ ID NO: 12, including the signal sequence, is shown below as SED ID NO: 19 (and corresponds to Genbank Accession No. CAD30563.1) 
MIGLALLMLLGAAAAVPREDGRIIGGQDCEPRSRPFMASLNYGYHFCGGVLINDQWVLSV
AHCWYNPYYMQVMLGEHDLRVFEGTEQLVKTNTIFWHEQYDYQTLDYDMMMIKLYHPVEV
TQSVAPISLPTGPPDGGMLCSVSGWGNMAMGEEVNLPTRLQCLDVPIVETVDCEAAYPGM
ISPRMVCAGYMDGGRDACNGDSGSPLVCEGVLTGLVSWGQGCALPNYPGVYVKVYEYLSW
IQTTLDANP
[SEQ ID NO: 19]
 (wherein the signal sequence and propeptide are underlined and in bold italics, respectively).

The trypsin ZT isoforms represented by SEQ ID NOs: 3 to 7 represent the active variants of these trypsins, *i.e.* variants that have been activated by cleavage of the N terminus of the trypsins. These trypsins are proteins expressed in the pyloric caeca / pancreas (pancreatic tissue in fish) with a number of amino acids on the N terminal end that are important for secretion out of the cells and for keeping the enzyme inactive.

For example, the full-length trypsin ZT isoforms are also disclosed herein as:
Uncleaved Atlantic cod trypsin ZT-1 isoform: 
MIGLALLMLLGAAAAAVPRDVGKIVGGHECEPNSRPFMASLNYGYHFCGGVLINDQWVLS
VAHCWYNPYYMQVMLGEHDLRVFEGTEQLVKTNTIFWHELYDYQTLDYDMMMIKLYHPVE
VTQSVAPISLPTGPPDGGMLCSVSGWGNMAMGEEVNLPTRLQCLDVPIVEPVACQASYPG
MISPRMMCVGFMDGGRDVCNGDSGSPLVCEGVLTGLVSWGRGCAEPNSPGVYVKVYEFLS
WIQTTLDANP
[SEQ ID NO: 20]
Uncleaved Atlantic cod trypsin ZT-2 isoform: 
MIGLALLMLLGAAAAAVPRDVGKIVGGHECEPNSRPFMASLNYGYHFCGGVLINDQWVLS
VAHCWYNPYYMQVMLGEHDLRVFEGTEQLVKTNTIFWHELYDYQTLDYDMMMIKLYHPVE
VTQSVAPISLPTGPPDGGMLCSVSGWGNMAMGEEVNLPTRLQCLDVPIVETVDCEAAYPG
MISPRMVCAGYMDGGRDACNGDSGSPLVCEGVLTGLVSWGQGCALPNYPGVYVKVYEYLS
WIQTTLDANP
[SEQ ID NO: 21]
Uncleaved Atlantic cod trypsin ZT-3 isoform: 
MIGLALLMLLGAAAAVPREDGRIIGGQDCEPRSRPFMASLNYGYHFCGGVLINDQWVLSV

AHCWYNPYYMQVMLGEHDLRVFEGTEQLVKTNTIFWHELYDYQTLDYDMMMIKLYHPVEV

TQSVAPISLPTGPPDGGMLCSVSGWGNMAMGEEVNLPTRLQCLDVPIVEPVACQASYPGM

ISPRMMCVGFMDGGRDVCNGDSGSPLVCEGVLTGLVSWGRGCAEPNSPGVYVKVYEFLSW

IQTTLDANP

[SEQ ID NO: 22]
Uncleaved Atlantic cod trypsin ZT-4 isoform:

MIGLALLMLLGAAAAVPREDGRIIGGQDCEPRSRPFMASLNYGYHFCGGVLINDQWVLSV
AHCWYNPYYMQVMLGEHDLRVFEGTEQLVKTNTIFWHELYDYQTLDYDMMMIKLYHPVEV
TQSVAPISLPTGPPDGGMLCSVSGWGNMAMGEEVNLPTRLQCLDVPIVETVDCEAAYPGM
ISPRMVCAGYMDGGRDACNGDSGSPLVCEGVLTGLVSWGQGCALPNYPGVYVKVYEYLSW
IQTTLDANP
[SEQ ID NO: 23]

The term 'amino acid' as used herein includes the standard twenty genetically-encoded amino acids and their corresponding stereoisomers in the 'D' form (as compared to the natural 'L' form), omega-amino acids and other naturally-occurring amino acids, unconventional amino acids (e.g., α,α-disubstituted amino acids, N-alkyl amino acids, *etc.)* and chemically derivatised amino acids (see below).

When an amino acid is being specifically enumerated, such as 'alanine' or 'Ala' or 'A', the term refers to both L-alanine and D-alanine unless explicitly stated otherwise. Other unconventional amino acids may also be suitable components for polypeptides of the present invention, as long as the desired functional property is retained by the polypeptide. For the peptides shown, each encoded amino acid residue, where appropriate, is represented by a single letter designation, corresponding to the trivial name of the conventional amino acid.

In accordance with convention, the amino acid sequences disclosed herein are provided in the N-terminus to C-terminus direction.

In one embodiment, the polypeptides of the invention comprise or consist of L-amino acids.

Persons of skill in the art will appreciate that the polypeptide having protease activity may comprise or consist of a fragment, variant, derivative or fusion thereof (or a fusion of said fragment, variant or derivative) of one of the above amino acid sequences, e.g. SEQ ID NOs: 1 to 12, provided that said fragment, variant, derivative or fusion retains (at least in part) the trypsin activity of said amino acid sequences.

Trypsin activity may be determined using methods well known in the art. For example, trypsin assay kits are commercially available from Abcam, Cambridge, UK (see Cat No. ab102531) and other suppliers. In one embodiment, trypsin activity is measured using Cbz-Gly-Pro-Arg-p-nitroanilide (Cbz-GPR-pNA) as a substrate (see EP 1,202,743 B and Stefansson et al., 2010, Comp Biochem Physiol B Biochem Mol Biol. 155(2):186-94).

Typically, the protease polypeptide has a specific activity of at least 1 U/mg of polypeptide, for example at least 10 U/mg, at least 50 U/mg, at least 100 U/mg, at least 200 U/mg or at least 500 U/mg. 'U' as used herein means an enzyme unit (one U is the amount of enzyme that catalyzes the conversion of 1 micro-mole of substrate per minute).

In one embodiment the polypeptide comprises or consists of a fragment of the amino acid sequence according to SEQ ID NO: 1, wherein the fragment exhibits protease activity.

Thus, where the polypeptide comprises an amino acid sequence according to any one of SEQ ID NOs: 1 to 12, it may comprise additional amino acids at its N- and/or C- terminus beyond those of SEQ ID NOs: 1 to 12. Likewise, where the polypeptide comprises a fragment, variant or derivative of an amino acid sequence according to SEQ ID NOs: 1 to 12, it may comprise additional amino acids at its N- and/or C- terminus.

Alternatively, the polypeptide having protease activity may correspond to a fragment of such a wildtype trypsin, such as SEQ ID NOs: 1 to 12, provided that said fragment retains (at least in part) the trypsin activity of the naturally occurring trypsin protein from which it is derived. Thus, the polypeptide may comprise or consist of at least 10 contiguous amino acids of SEQ ID NOs: 1 to 12, e.g. at least 15,16, 17, 18, 19, 20, 30, 40, 50, 60, 70, 80, 90, 100, 110, 120, 130, 140, 150, 160, 170, 180, 190, 200, 210, 220, 230 or 240 contiguous amino acids any one of SEQ ID NOs: 1 to 12.

For example, the fragment may comprise or consist of amino acid residues 61 to 77 of any one of SEQ ID NOs:1 to 12. Alternatively, or in addition, the fragment may comprise or consist of amino acid residues 225 to 241 of any one of SEQ ID NOs: 1 to 12.

It will be appreciated by persons skilled in the art that the polypeptide of the invention may alternatively comprise or consist of a variant of the amino acid sequence according to any one of SEQ ID NOs: 1 to 12 (or fragments thereof). Such a variant may be a non-naturally occurring variant.

By 'variants' of the polypeptide we include insertions, deletions and substitutions, either conservative or non-conservative. In particular, we include variants of the polypeptide where such changes retain, at least in part, the trypsin activity of the said polypeptide.

Such variants may be made using the methods of protein engineering and site-directed mutagenesis well known in the art using the recombinant polynucleotides (see Molecular Cloning: a Laboratory Manual, 3rd edition, Sambrook & Russell, 2000, Cold Spring Harbor Laboratory Press) (8).

In one embodiment, the variant has an amino acid sequence which has at least 50% identity with the amino acid sequence according to any one of SEQ ID NOs: 1 to 12, or a fragment thereof, for example at least 55%, 60%, 65%, 70%, 75%, 80%, 90%, 95%, 96%, 97%, 98% or at least 99% identity.

The percent sequence identity between two polypeptides may be determined using suitable computer programs, for example the GAP program of the University of Wisconsin Genetic Computing Group and it will be appreciated that percent identity is calculated in relation to polypeptides whose sequences have been aligned optimally.

The alignment may alternatively be carried out using the Clustal W program (as described in Thompson et al., 1994, Nuc. Acid Res. 22:4673-4680)(9).

The parameters used may be as follows:
Fast pairwise alignment parameters: K-tuple(word) size; 1, window size; 5, gap penalty; 3, number of top diagonals; 5. Scoring method: x percent.
Multiple alignment parameters: gap open penalty; 10, gap extension penalty; 0.05. Scoring matrix: BLOSUM.
Alternatively, the BESTFIT program may be used to determine local sequence alignments.

In one embodiment, the polypeptide having protease activity is a variant of SEQ ID NO:1 or 2 comprising one or more mutated amino acids selected from the group consisting of amino acid positions:
E21, H25, H29, V47, K49, D50, L63, H71, H72, R74, N76, T79, Y82, S85, S87, S89, N98, I99, V121, M135, V138, M145, V148, D150, K154, L160, M175, S179, A183, L185, V212, Y217, P225, A229, V233, L234, V238, N240, Y241 and/or M242.
or a fragment thereof which exhibits protease activity (wherein the amino acid sequence and numbering is according to Protein Data Bank [PDB] entry '2EEK!', with the initial isoleucine of SEQ ID NO: 1 or 2 being numbered as position I-16).

Thus, the polypeptide having protease activity may be a variant of SEQ ID NO:1 or 2 comprising one or more amino acids mutations selected from the group consisting of:
E21T, H25Y, H29(Y/N), V47I, K49E, D50Q, L63I, H71D, H72N, R74(K/E), N76(T/L), T79(S/N), Y82F, S85A, S87(K/R), S89R, N98T, I99L, V121I, M135Q, V138I, M145(T/L/V/E/K), V148G, D150S, K154(T/V), L160(I/A), M175(K/Q), S179N, A183V, L185G, V212I, Y217(D/H/S), P225Y, A229V, V233N, L234Y, V238I, N240S, Y241N and/or M242I;
or a fragment thereof which exhibits protease activity.

In one embodiment, the polypeptide having protease activity may comprise or consist of the amino acid sequence of SEQ ID NO:1 or 2 with one of the following defined mutations in Table 1 (or combinations thereof).

**Table 1**

| Sequences of exemplary trypsin polypeptides | |
|---|---|
| ***Polypeptide name*** | ***Mutations relative to SEQ ID NO:1 or 2**** |
| EZA-001 | (none) |
| EZA-002 | N240S, Y241N, S87K |
| EZA-003 | K154T |
| EZA-004 | K154L |
| EZA-005 | K154V |
| EZA-006 | K154E |
| EZA-007 | N98T |
| EZA-008 | I99L |
| EZA-009 | L185G, P225Y |
| EZA-010 | V212I |
| EZA-011 | Y217D, M175K |
| EZA-012 | Y217H |
| EZA-013 | Y217S |
| EZA-014 | A229V |
| EZA-015 | H25Y |
| EZA-016 | H25N |
| EZA-017 | H29Y |
| EZA-018 | H71D |
| EZA-019 | H72N |
| EZA-020 | R74K |
| EZA-021 | R74E |
| EZA-022 | N76T |
| EZA-023 | N76L, Y82F |
| EZA-024 | T79S |
| EZA-025 | T79N |
| EZA-026 | K49E, D50Q |
| EZA-027 | S87R |
| EZA-028 | E21T, H71D, D150S, K154V |
| EZA-029 | S179N,V233N |
| EZA-030 | M135Q |
| EZA-031 | M145K, V148G |
| EZA-032 | M175Q |
| EZA-033 | L63I, S85A |
| EZA-034 | L160I |
| EZA-035 | V138I, L160A, A183V |
| EZA-036 | V121I |
| EZA-037 | V47I, V238I, M242I |
| EZA-038 | V238I |
| EZA-039 | L234Y |

Likewise, the polypeptide having protease activity may comprise or consist of the amino acid of SEQ ID NO:1 or 2 with one of the following defined mutations (or combinations thereof):
(a) H25N, N76T
(b) H25N, H29Y
(c) H25N, M135Q
(d) H29Y, T79N, M135Q
(e) I99L, V121I, L160I, Y217H
(f) V121I, L160I
(g) H72N, R74E, S87K
(h) H25N, M135Q, Y217H
(i) T79N, V121I, V212I
(j) H29Y, N76T, I99L, M135Q
(k) K49E, D50Q, N76L, Y82F, S179N, V233N
(l) M145K, V148G, N76L, Y82F, S179N, V233N
(m) H25N, N76T, S87K, K154T
(n) H25Q
(o) H25D
(p) H25S
(q) K24E, H25N
(r) Y97N
(s) N100D
(t) A120S, A122S
(u) M135E
(v) V204Q, A122S
(w) T79D
(x) R74D
(y) K49E
(z) K49S,D50Q
(aa) D50Q
(bb) Q178D
(cc) S87R

In one preferred embodiment, the polypeptide having protease activity is a variant of the amino acid sequence of SEQ ID NO:1 or 2 which does not comprise histidine at position 25.

For example, the polypeptide having protease activity may comprise or consist of the amino acid sequence of SEQ ID NO:3 (comprising an H25N mutation; see box in sequence below):

16
IVGGYECTKNSQAHQVSLNSGYHFCGGSLVSKDWVVSAAHCYKSVLRVRLGEHHIRVNEG

79
TEQYISSSSVIRHPNYSSYNINNDIMLIKLTKPATLNQYVHAVALPTECAADAMCTVSG

141
WGNTMSSVADGDKLQCLSLPILSHADCANSYPGMITQSMFCAGYLEGGKDSCQGDSGGPV

200
VCNGVLQGVVSWGYGCAERDHPGVYAKVCVLSGWVRDTMANY

[SEQ ID NO: 24]

In an alternative preferred embodiment, the polypeptide having protease activity is a variant of the amino acid sequence of SEQ ID NO:1 or 2 which does not comprise lysine at position 160.

For example, the polypeptide having protease activity may comprise or consist of the amino acid sequence of SEQ ID NO: 4 (comprising an L160I mutation; see box in sequence below):

16
IVGGYECTKHSQAHQVSLNSGYHFCGGSLVSKDWVVSAAHCYKSVLRVRLGEHHIRVNEG

79
TEQYISSSSVIRHPNYSSYNINNDIMLIKLTKPATLNQYVHAVALPTECAADAMCTVSG

141
WGNTMSSVADGDKLQCLSIPILSHADCANSYPGMITQSMFCAGYLEGGKDSCQGDSGGPV

200
VCNGVLQGVVSWGYGCAERDHPGVYAKVCVLSGWVRDTMANY

[SEQ ID NO: 25]

It will be appreciated by persons skilled in the art that the above identified mutations (defined by reference to the amino acid sequence of trypsin I of Atlantic cod, SEQ ID NO:1 or 2) could also be made in trypsins from other species. For example, the specific mutations highlighted in SEQ ID NOS: 3 and 4 (H25N and L160I, respectively) could be made in the trypsin from Alaskan Pollock (for example see GenBank: BAH70476.3, wherein the amino acid sequence of the active trypsin commences at position I20, such that H25 corresponds to H29 in BAH70476.3, *etc*).

In a further embodiment of the first aspect of the invention, the polypeptide comprises or consists of a fusion protein.

By 'fusion' of a polypeptide we include an amino acid sequence corresponding to a polypeptide having protease activity (such as SEQ ID NOS: 1 to 12 or a fragment or variant thereof) fused to any other polypeptide. For example, the said polypeptide may be fused to a polypeptide such as glutathione-S-transferase (GST) or protein A in order to facilitate purification of said polypeptide. Examples of such fusions are well known to those skilled in the art. Similarly, the said polypeptide may be fused to an oligo-histidine tag such as His6 or to an epitope recognised by an antibody such as the well-known Myc tag epitope. Fusions to any variant or derivative of said polypeptide are also included in the scope of the invention.

The fusion may comprise a further portion which confers a desirable feature on the said polypeptide of the invention; for example, the portion may be useful in augmenting or prolonging the therapeutic effect. For example, in one embodiment the fusion comprises human serum albumin or a similar protein.

Alternatively, the fused portion may be, for example, a biotin moiety, a radioactive moiety, a fluorescent moiety, for example a small fluorophore or a green fluorescent protein (GFP) fluorophore, as well known to those skilled in the art. The moiety may be an immunogenic tag, for example a Myc tag, as known to those skilled in the art or may be a lipophilic molecule or polypeptide domain that is capable of promoting cellular uptake of the polypeptide, as known to those skilled in the art.

In a further embodiment of the first aspect of the invention, the polypeptide, or fragment, variant, fusion or derivative thereof, comprises or consists of one or more amino acids that are modified or derivatised.

Chemical derivatives of one or more amino acids may be achieved by reaction with a functional side group. Such derivatised molecules include, for example, those molecules in which free amino groups have been derivatised to form amine hydrochlorides, p-toluene sulphonyl groups, carboxybenzoxy groups, *t*-butyloxycarbonyl groups, chloroacetyl groups or formyl groups. Free carboxyl groups may be derivatised to form salts, methyl and ethyl esters or other types of esters and hydrazides. Free hydroxyl groups may be derivatised to form O-acyl or O-alkyl derivatives. Also included as chemical derivatives are those peptides which contain naturally occurring amino acid derivatives of the twenty standard amino acids. For example: 4-hydroxyproline may be substituted for proline; 5-hydroxylysine may be substituted for lysine; 3-methylhistidine may be substituted for histidine; homoserine may be substituted for serine and ornithine for lysine. Derivatives also include peptides containing one or more additions or deletions as long as the requisite activity is maintained. Other included modifications are amidation, amino terminal acylation (e.g. acetylation or thioglycolic acid amidation), terminal carboxylamidation (e.g. with ammonia or methylamine), and the like terminal modifications.

It will be further appreciated by persons skilled in the art that peptidomimetic compounds may also be useful. Thus, by 'polypeptide' we include peptidomimetic compounds which have an anti-inflammatory activity of the polypeptide of any of SEQ ID NOS: 1 to 12. The term 'peptidomimetic' refers to a compound that mimics the conformation and desirable features of a particular peptide as a therapeutic agent.

For example, the polypeptides used in the invention include not only molecules in which amino acid residues are joined by peptide (-CO-NH-) linkages but also molecules in which the peptide bond is reversed. Such retro-inverso peptidomimetics may be made using methods known in the art, for example such as those described in Meziere et al. (1997) J. Immunol. 159:3230-3237) (10). This approach involves making pseudopeptides containing changes involving the backbone, and not the orientation of side chains. Retro-inverse peptides, which contain NH-CO bonds instead of CO-NH peptide bonds, are much more resistant to proteolysis. Alternatively, the polypeptide of the invention may be a peptidomimetic compound wherein one or more of the amino acid residues are linked by a -y(CH₂NH)- bond in place of the conventional amide linkage.

In a further alternative, the peptide bond may be dispensed with altogether provided that an appropriate linker moiety which retains the spacing between the carbon atoms of the amino acid residues is used; it may be advantageous for the linker moiety to have substantially the same charge distribution and substantially the same planarity as a peptide bond.

It will be appreciated that the polypeptide may conveniently be blocked at its N- or C-terminus so as to help reduce susceptibility to exoproteolytic digestion.

A variety of uncoded or modified amino acids such as D-amino acids and N-methyl amino acids have also been used to modify polypeptides. In addition, a presumed bioactive conformation may be stabilised by a covalent modification, such as cyclisation or by incorporation of lactam or other types of bridges, for example see Veber et al., 1978, Proc. Natl. Acad. Sci. USA 75:2636 and Thorsett et al., 1983, Biochem. Biophys. Res. Comm. 111:166 (11,12).

In one preferred embodiment, however, the polypeptide comprises one or more amino acids modified or derivatised by PEGylation, amidation, esterification, acylation, acetylation and/or alkylation.

It will be appreciated by persons skilled in the art that the polypeptides may be of any suitable length. Preferably, the polypeptides are between 10 and 30 amino acids in length, for example between 10 and 20, 12 and 18, 12 and 16, or 15 and 20 amino acids in length. Alternatively, the polypeptide may be between 150 and 250 amino acids in length, for example between 200 and 250, 210 and 240, 220 and 230, or 220 and 225 amino acids in length.

In one embodiment, the polypeptide is linear.

In a further embodiment, the polypeptide is a recombinant polypeptide.

The polypeptides of the invention, as well as nucleic acid molecules, vectors and host cells for producing the same, may be made using methods well known in the art (for example, see Green & Sambrook, 2012, Molecular Cloning, A Laboratory Manual, Fourth Edition, Cold Spring Harbor, New York)(8).

Recombinant methods for producing polypeptides having protease activity, such as trypsins, are disclosed in WO 2015/150799 to Enzymatica.

Alternatively, the polypeptides may be synthesised by known means, such as liquid phase and solid phase synthesis (for example, t-Boc solid-phase peptide synthesis and BOP-SPPS).

It will be appreciated by persons skilled in the art that the present invention also includes pharmaceutically acceptable acid or base addition salts of the above described polypeptides. The acids which are used to prepare the pharmaceutically acceptable acid addition salts of the aforementioned base compounds useful in this invention are those which form non-toxic acid addition salts, *i.e.* salts containing pharmacologically acceptable anions, such as the hydrochloride, hydrobromide, hydroiodide, nitrate, sulphate, bisulphate, phosphate, acid phosphate, acetate, lactate, citrate, acid citrate, tartrate, bitartrate, succinate, maleate, fumarate, gluconate, saccharate, benzoate, methanesulphonate, ethanesulphonate, benzenesulphonate, p-toluenesulphonate and pamoate [*i.e.* 1,1'-methylene-bis-(2-hydroxy-3 naphthoate)] salts, among others.

Pharmaceutically acceptable base addition salts may also be used to produce pharmaceutically acceptable salt forms of the polypeptides. The chemical bases that may be used as reagents to prepare pharmaceutically acceptable base salts of the present compounds that are acidic in nature are those that form non-toxic base salts with such compounds. Such non-toxic base salts include, but are not limited to those derived from such pharmacologically acceptable cations such as alkali metal cations (e.g. potassium and sodium) and alkaline earth metal cations (e.g. calcium and magnesium), ammonium or water-soluble amine addition salts such as N-methylglucamine-(meglumine), and the lower alkanolammonium and other base salts of pharmaceutically acceptable organic amines, among others.

It will be further appreciated that the polypeptides may be lyophilised for storage and reconstituted in a suitable carrier prior to use. Any suitable lyophilisation method (e.g. spray drying, cake drying) and/or reconstitution techniques can be employed. It will be appreciated by those skilled in the art that lyophilisation and reconstitution can lead to varying degrees of activity loss and that use levels may have to be adjusted upward to compensate. Preferably, the lyophilised (freeze dried) polypeptide loses no more than about 20%, or no more than about 25%, or no more than about 30%, or no more than about 35%, or no more than about 40%, or no more than about 45%, or no more than about 50% of its activity (prior to lyophilisation) when rehydrated.

The compounds of the invention are typically provided in the form of a therapeutic composition, in which the polypeptide is formulated together with a pharmaceutically acceptable buffer, diluent, carrier, adjuvant or excipient. Additional compounds may be included in the compositions, including, chelating agents such as EDTA, citrate, EGTA or glutathione. The antiviral/therapeutic compositions may be prepared in a manner known in the art that is sufficiently storage stable and suitable for administration to humans and animals. The therapeutic compositions may be lyophilised, e.g., through freeze drying, spray drying, spray cooling, or through use of particle formation from supercritical particle formation.

By "pharmaceutically acceptable" we mean a non-toxic material that does not decrease the effectiveness of the trypsin activity of the compounds of the invention. Such pharmaceutically acceptable buffers, carriers or excipients are well-known in the art (see Remington's Pharmaceutical Sciences, 18th edition, A.R Gennaro, Ed., Mack Publishing Company (1990) and handbook of Pharmaceutical Excipients, 3rd edition, A. Kibbe, Ed .,

Pharmaceutical Press (2000). (13, 14).

The term "buffer" is intended to mean an aqueous solution containing an acid-base mixture with the purpose of stabilising pH. Examples of buffers are Trizma, Bicine, Tricine, MOPS, MOPSO, MOBS, Tris, Hepes, HEPBS, MES, phosphate, carbonate, acetate, citrate, glycolate, lactate, borate, ACES, ADA, tartrate, AMP, AMPD, AMPSO, BES, CABS, cacodylate, CHES, DIPSO, EPPS, ethanolamine, glycine, HEPPSO, imidazole, imidazolelactic acid, PIPES, SSC, SSPE, POPSO, TAPS, TABS, TAPSO and TES.

The term "diluent" is intended to mean an aqueous or non-aqueous solution with the purpose of diluting the peptide in the therapeutic preparation. The diluent may be one or more of saline, water, polyethylene glycol, propylene glycol, ethanol or oils (such as safflower oil, corn oil, peanut oil, cottonseed oil or sesame oil).

The term "adjuvant" is intended to mean any compound added to the formulation to increase the biological effect of the polypeptide of the invention. The adjuvant may be one or more of zinc, copper or silver salts with different anions, for example, but not limited to fluoride, chloride, bromide, iodide, tiocyanate, sulfite, hydroxide, phosphate, carbonate, lactate, glycolate, citrate, borate, tartrate, and acetates of different acyl composition. The adjuvant may also be cationic polymers such as cationic cellulose ethers, cationic cellulose esters, deacetylated hyaluronic acid, chitosan, cationic dendrimers, cationic synthetic polymers such as poly(vinyl imidazole), and cationic polypeptides such as polyhistidine, polylysine, polyarginine, and peptides containing these amino acids.

The excipient may be one or more of carbohydrates, polymers, lipids and minerals. Examples of carbohydrates include lactose, glucose, sucrose, mannitol, and cyclodextrines, which are added to the composition, e.g., for facilitating lyophilisation. Examples of polymers are starch, cellulose ethers, cellulose carboxymethylcellulose, hydroxypropylmethyl cellulose, hydroxyethyl cellulose, ethylhydroxyethyl cellulose, alginates, carageenans, hyaluronic acid and derivatives thereof, polyacrylic acid, polysulphonate, polyethylenglycol/polyethylene oxide, polyethyleneoxide/polypropylene oxide copolymers, polyvinylalcohol/polyvinylacetate of different degree of hydrolysis, and polyvinylpyrrolidone, all of different molecular weight, which are added to the composition, e.g., for viscosity control, for achieving bioadhesion, or for protecting the lipid from chemical and proteolytic degradation. Examples of lipids are fatty acids, phospholipids, mono-, di-, and triglycerides, ceramides, sphingolipids and glycolipids, all of different acyl chain length and saturation, egg lecithin, soy lecithin, hydrogenated egg and soy lecithin, which are added to the composition for reasons similar to those for polymers. Examples of minerals are talc, magnesium oxide, zinc oxide and titanium oxide, which are added to the composition to obtain benefits such as reduction of liquid accumulation or advantageous pigment properties.

In one embodiment, the compound may be provided together with a stabiliser, such as calcium chloride.

The polypeptides of the invention may be formulated into any type of therapeutic composition known in the art to be suitable for the delivery of polypeptide agents.

In one embodiment, the compounds may simply be dissolved in water, saline, polyethylene glycol, propylene glycol, ethanol or oils (such as safflower oil, corn oil, peanut oil, cottonseed oil or sesame oil), tragacanth gum, and/or various buffers.

In a further embodiment, the compounds of the invention may be in the form of a liposome, in which the polypeptide is combined, in addition to other pharmaceutically acceptable carriers, with amphipathic agents such as lipids, which exist in aggregated forms as micelles, insoluble monolayers and liquid crystals. Suitable lipids for liposomal formulation include, without limitation, monoglycerides, diglycerides, sulfatides, lysolecithin, phospholipids, saponin, bile acids, and the like. Suitable lipids also include the lipids above modified by poly(ethylene glycol) in the polar headgroup for prolonging bloodstream circulation time. Preparation of such liposomal formulations is can be found in for example US 4,235,871.

The therapeutic compounds of the invention may also be in the form of biodegradable microspheres. Aliphatic polyesters, such as poly(lactic acid) (PLA), poly(glycolic acid) (PGA), copolymers of PLA and PGA (PLGA) or poly(caprolactone) (PCL), and polyanhydrides have been widely used as biodegradable polymers in the production of microspheres. Preparations of such microspheres can be found in US 5,851,451 and in EP 0 213303.

In a further embodiment, the compounds of the invention are provided in the form of polymer gels, where polymers such as starch, cellulose ethers, cellulose carboxymethylcellulose, hydroxypropylmethyl cellulose, hydroxyethyl cellulose, ethylhydroxyethyl cellulose, alginates, carageenans, hyaluronic acid and derivatives thereof, polyacrylic acid, polyvinyl imidazole, polysulphonate, polyethylenglycol/polyethylene oxide, polyethyleneoxide/polypropylene oxide copolymers, polyvinylalcohol/polyvinylacetate of different degree of hydrolysis, and polyvinylpyrrolidone are used for thickening of the solution containing the peptide. The polymers may also comprise gelatin or collagen.

It will be appreciated that the compounds of the invention may include ions and a defined pH for potentiation of action of the polypeptides. Additionally, the compositions may be subjected to conventional therapeutic operations such as sterilisation and/or may contain conventional adjuvants such as preservatives, stabilisers, wetting agents, emulsifiers, buffers, fillers, *etc.*

In one preferred embodiment, the compound is formulated in a Tris or phosphate buffer, together with one or more of EDTA, xylitol, sorbitol, propylene glycol and glycerol.

The compounds according to the invention and therapeutic compositions thereof may be administered via any suitable route known to those skilled in the art. Thus, possible routes of administration include oral, buccal, parenteral (intravenous, subcutaneous, and intramuscular), topical, ocular, nasal, pulmonar, parenteral, vaginal and rectal. Also administration from implants is possible.

In one preferred embodiment, the therapeutic compositions are administered topically, in a form suitable for delivery to the oropharynx. For example, the polypeptide may be formulated as a mouth spray, lozenge, pastille, tablet, syrup or chewing gum.

In an alternative embodiment, the therapeutic compositions are administered parenterally, for example, intravenously, intracerebroventricularly, intraarticularly, intra-arterially, intraperitoneally, intrathecally, intraventricularly, intrasternally, intracranially, intramuscularly or subcutaneously, or they may be administered by infusion techniques. They are conveniently used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

The therapeutic compositions will be administered to a patient in a pharmaceutically effective dose. A 'therapeutically effective amount', or 'effective amount', or 'therapeutically effective', as used herein, refers to that amount which provides a therapeutic effect for a given condition and administration regimen. This is a predetermined quantity of active material calculated to produce a desired therapeutic effect in association with the required additive and diluent, *i.e.* a carrier or administration vehicle. Further, it is intended to mean an amount sufficient to reduce and most preferably prevent, a clinically significant deficit in the activity, function and response of the host. Alternatively, a therapeutically effective amount is sufficient to cause an improvement in a clinically significant condition in a host. As is appreciated by those skilled in the art, the amount of a compound may vary depending on its specific activity. Suitable dosage amounts may contain a predetermined quantity of active composition calculated to produce the desired therapeutic effect in association with the required diluent. In the methods and use for manufacture of compositions of the invention, a therapeutically effective amount of the active component is provided. A therapeutically effective amount can be determined by the ordinary skilled medical or veterinary worker based on patient characteristics, such as age, weight, sex, condition, complications, other diseases, *etc.,* as is well known in the art. The administration of the pharmaceutically effective dose can be carried out both by single administration in the form of an individual dose unit or else several smaller dose units and also by multiple administrations of subdivided doses at specific intervals. Alternatively, the dose may be provided as a continuous infusion over a prolonged period.

An exemplary therapeutic composition of the invention is described in the Examples below.

In one embodiment the polypeptide can be provided in a delivery device, for example in a spray container, which may be configured for ease of delivery to the oropharynx.

In one embodiment the polypeptide is for use in combination with one or more additional active agents.

For example, the additional active agents may be selected from the group consisting of antimicrobial agents (including antibiotics, antiviral agents and anti-fungal agents), anti-inflammatory agents (including steroids and non-steroidal anti-inflammatory agents) and antiseptic agents.

In one embodiment the active agents are one or more antimicrobial agents, for example antibiotics selected from the group consisting of penicillins, cephalosporins, fluoroquinolones, aminoglycosides, monobactams, carbapenems and macrolides.

For example, the antibiotics may be selected from the group consisting of amikacin, amoxicillin, ampicillin, azithromycin, carbenicillin, carbapenems, cefotaxime, ceftazidime, ceftriaxone, cefuroxime, cephalosporins, chloramphenicol, ciprofloxacin, clindamycin, dalacin, dalfopristin, daptomycin, doxycycline, enrofloxacin, ertapenem, erythromycin, fluoroquinolones, gentamicin, marbofloxacin, meropenem, metronidazole, minocycline, moxifloxacin, nafcillin, ofloxacin, oxacillin, penicillin, quinupristin, rifampin, silver sulfadiazine, sulfamethoxazole, teicoplanin, tetracycline, tobramycin, trimethoprim, vancomycin, bacitracin and polymyxin B, or a mixture thereof.

In one embodiment, the additional antibiotics may be for topical or oral administration.

A second related aspect of the invention provides the use of a compound according to the first aspect of the invention in the preparation of a medicament for the treatment or prevention of a rhinovirus infection in a mammal.

### Advantageously, the compound is benzoic acid 4-hydroxy propyl ester

In one embodiment, the medicament further comprises a polypeptide having protease activity. The polypeptide having protease activity may comprise or consist of an amino acid sequence of SEQ ID NO: 1 or 2, or a fragment, variant, derivative or fusion thereof (or a fusion of said fragment, variant or derivative) which retains the trypsin activity of said amino acid sequence. For example, the polypeptide may be a trypsin from Atlantic cod, for example trypsin I or trypsin ZT.

A related, third aspect of the invention provides a method for the treatment or prevention of a rhinovirus infection in a mammal comprising administering to the subject a therapeutically-effective amount of a compound according to the first aspect of the invention.

### Advantageously, the compound is benzoic acid 4-hydroxy propyl ester

In one embodiment, the method further comprises administering a polypeptide having protease activity.

The polypeptide having protease activity may comprise or consist of an amino acid sequence of SEQ ID NO: 1 or 2, or a fragment, variant, derivative or fusion thereof (or a fusion of said fragment, variant or derivative) which retains the trypsin activity of said amino acid sequence. For example, the polypeptide may be a trypsin from Atlantic cod, for example trypsin I or trypsin ZT.

Preferences and options for a given aspect, feature or parameter of the invention should, unless the context indicates otherwise, be regarded as having been disclosed in combination with any and all preferences and options for all other aspects, features and parameters of the invention. For example, in one embodiment the invention provides a polypeptide consisting of an amino acid sequence of any one of SEQ ID NO: 1 to 7 for use in the treatment of a coronavirus infection in a human.

The listing or discussion of an apparently prior-published document in this specification should not necessarily be taken as an acknowledgement that the document is part of the state of the art or is common general knowledge.

The use of the word "a" or "an" when used in conjunction with the term "comprising" in the claims and/or the specification may mean "one," but it is also consistent with the meaning of "one or more," "at least one," and "one or more than one."

These, and other, embodiments of the invention will be better appreciated and understood when considered in conjunction with the above description. It should be understood, however, that the above description, while indicating various embodiments of the invention and numerous specific details thereof, is given by way of illustration and not of limitation. Many substitutions, modifications, additions and/or rearrangements may be made within the scope of the invention without departing from the spirit thereof, and the invention includes all such substitutions, modifications, additions and/or rearrangements. Preferred, non-limiting examples which embody certain aspects of the invention will now be described.

The listing or discussion of an apparently prior-published document in this specification should not necessarily be taken as an acknowledgement that the document is part of the state of the art or is common general knowledge.

Preferred, non-limiting examples which embody certain aspects of the invention will now be described, with reference to the following figures:
**Figure 1****.** Schematic representation of assay protocol
**Figure 2****.** Effect of pre-treatment only with benzoic acid 4-hydroxy propyl ester ("placebo") and PBS negative control ("PBS") on rhinovirus RV16 titre.
**Figure 3****.** Effect of combined pre-treatment and post-treatment with benzoic acid 4-hydroxy propyl ester ("placebo") and PBS negative control ("PBS") on rhinovirus RV16 titre.

### EXAMPLES

### Materials & Methods

### Test formulation

| ***Component*** | ***Amount (w*/*w%)*** |
|---|---|
| Purified water | 99.68 |
| Disodium phosphate dihydrate | 0.047 |
| Sodium dihydrogen phosphate monohydrate | 0.101 |
| Benzoic acid 4-hydroxy propyl ester (propagin; propyl paraben; N-propyl-p-hydroxy-benzoate) | 0.03 |
| Sucralose, ≥ 98% | 0.06 |
| Menthol, ≥ 99% | 0.0042 |
| Ethanol, 96% | 0.08 |
| 6 M HCI | (used to adjust solution to pH 6.4) |
| 6 M NaOH | |

### Assay protocol

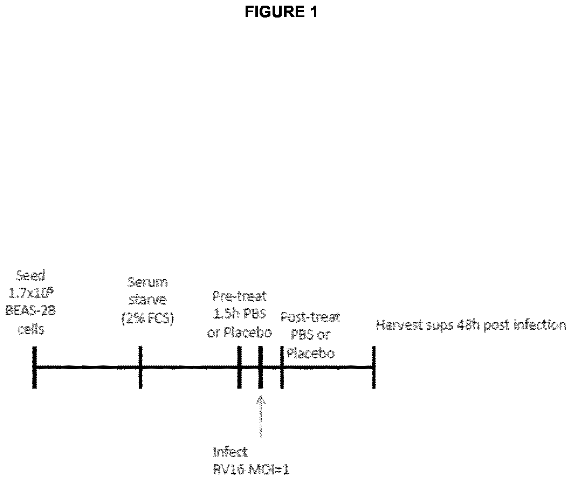

- BEAS-2B cells were seeded into 12 well plates at 1.7x10⁵ cells per well and left to grow for 24h in 10% foetal calf serum (FCS) containing RPMI medium
- BEAS-2B cells were then placed into 2% FCS containing RPMI medium
- Placebo was sprayed into a 10 mL universal tube, and this was used to produce dilutions of ½, 1/5 and 1/10 in 2% FCS containing RPMI medium.
- Phosphate buffered saline (PBS) pH7.4, was used as a comparison (negative control) and diluted in the same way.
- 1 mL per well was added onto each well and incubated for 90 mins (pre-treatment)
- The supernatant containing test formulation or PBS was removed, and BEAS-2B cells were then infected with MOI of 1 RV16, at room temperature for 1h with shaking.
- RV16 was washed off 3X in 2% FCS containing RPMI medium
- For pre-treatment, 2% FCS containing medium was then added 1 mL per well.
- For pre- and post-treatment, the test formulation and PBS control were diluted fresh as above and added 1 mL per well.
- Cells were incubated for 48h and then supernatants frozen at -20°C.
- Supernatants were thawed and serially diluted 10-fold and used in titration of HeLa cells in replicates of 8, using standard lab protocols. All plates were read at 4 days post-infection
- See Figure 1

### Results

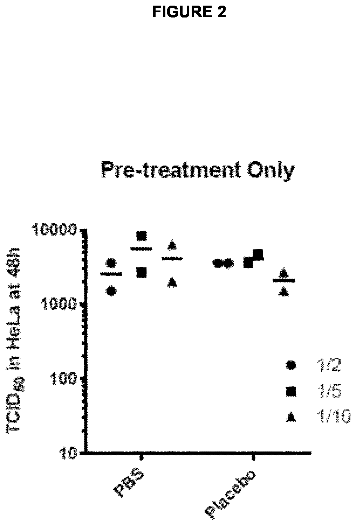

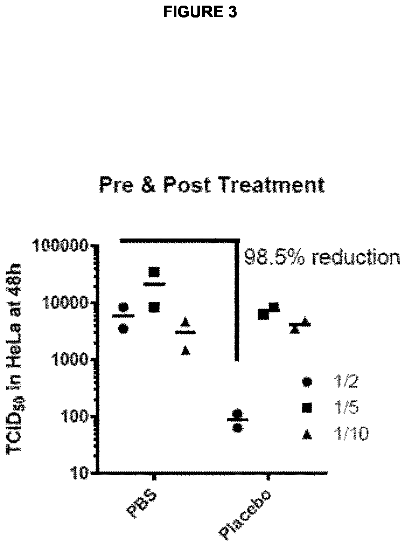

- BEAS-2B cells exhibited no obvious deleterious effects following pre-treatment, or pre- and post-treatment, with the test formulation and PBS control at any dilution
- Pre-treatment only appeared to have had no effect on RV16 titre (see Figure 2)
- For pre- and post-treatment, the test formulation at ½ dilution reduced RV16 titre by 98.5% (see Figure 3)
- The test formulation at 1/5 dilution reduced RV16 titre by 68%
- No effect was seen at 1/10 dilution of the test formulation.

### Conclusions

- The data indicate that the test formulation inhibits rhinovirus replication

## Claims

1. A compound of formula I for use in the treatment or prevention of a rhinovirus infection in a mammal 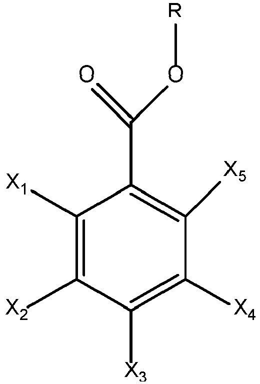 wherein:
R represents a C₁₋₁₀ alkyl group;
X₁, X₂, X₃, X₄ and X₅ independently represent -H or -OH; and
and wherein at least one of X₁, X₂, X₃, X₄ and X₅ is -OH.

2. A compound for use according to Claim 1 wherein the infection is in the upper respiratory tract.

3. A compound for use according to Claim 1 or 2 wherein the infection is a common cold.

4. A compound for use according to any one of the preceding claims wherein the rhinovirus is human rhinovirus.

5. A compound for use according to any one of the preceding claims wherein the rhinovirus is
Type A and is selected from the group consisting of human rhinovirus serotypes HRV-A1, HRV-A2, HRV-A7, HRV-A8, HRV-A9, HRV-A10, HRV-A11, HRV-A12, HRV-A13, HRV-A15, HRV-A16, HRV-A18, HRV-A19, HRV-A20, HRV-A21, HRV-A22, HRV-A23, HRV-A24, HRV-A25, HRV-A28, HRV-A29, HRV-A30, HRV-A31, HRV-A32, HRV-A33, HRV-A34, HRV-A36, HRV-A38, HRV-A39, HRV-A40, HRV-A41, HRV-A43, HRV-A44, HRV-A45, HRV-A46, HRV-A47, HRV-A49, HRV-A50, HRV-A51, HRV-A53, HRV-A54, HRV-A55, HRV-A56, HRV-A57, HRV-A58, HRV-A59, HRV-A60, HRV-A61, HRV-A62, HRV-A63, HRV-A64, HRV-A65, HRV-A66, HRV-A67, HRV-A68, HRV-A71, HRV-A73, HRV-A74, HRV-A75, HRV-A76, HRV-A77, HRV-A78, HRV-A80, HRV-A81, HRV-A82, HRV-A85, HRV-A88, HRV-A89, HRV-A90, HRV-A94, HRV-A95, HRV-A96, HRV-A98, HRV-A100, HRV-A101, HRV-A102 and HRV-A103, or is human rhinovirus.
or wherein the rhinovirus is
Type B and is selected from the group consisting of human rhinovirus serotypes HRV-B3, HRV-B4, HRV-B5, HRV-B6, HRV-B14, HRV-B17, HRV-B26, HRV-B27, HRV-B35, HRV-B37, HRV-B42, HRV-B48, HRV-B52, HRV-B69, HRV-B70, HRV-B72, HRV-B79, HRV-B83, HRV-B84, HRV-B86, HRV-B91, HRV-B92, HRV-B93, HRV-B97 and HRV-B99, or is human rhinovirus.
or wherein the rhinovirus is
Type C and is selected from the group consisting of human rhinovirus serotypes HRV-C1, HRV-C2, HRV-C3, HRV-C4, HRV-C5, HRV-C6, HRV-C7, HRV-C8, HRV-C9, HRV-C10, HRV-C11, HRV-C12, HRV-C13, HRV-C14, HRV-C15, HRV-C16, HRV-C17, HRV-C18, HRV-C19, HRV-C20, HRV-C21, HRV-C22, HRV-C23, HRV-C24, HRV-C25, HRV-C26, HRV-C27, HRV-C28, HRV-C29, HRV-C30, HRV-C31, HRV-C32, HRV-C33, HRV-C34, HRV-C35, HRV-C36, HRV-C37, HRV-C38,HRV-C39, HRV-C40, HRV-C41, HRV-C42, HRV-C43, HRV-C44, HRV-C45, HRV-C46, HRV-C47, HRV-C48, HRV-C49, HRV-C50 and HRV-C51.

6. A compound for use according to any one of the preceding claims wherein R represents an unsubstituted alkyl group.

7. A compound for use according to any one of the preceding claims wherein R represents a C₁₋₈ alkyl group, for example a C₁₋₆ alkyl group, a C₁₋₅ alkyl group, a C₁₋₄ alkyl group, a C₁₋₃ alkyl group, a C₁₋₂ alkyl group or a C₁ alkyl group.

8. A compound for use according to any one of the preceding claims wherein R represents a methyl group, an ethyl group, a propyl group or a butyl group.

9. A compound for use according to any one of the preceding claims wherein R represents -(CH₂)ₙCH₃, wherein 'n' is an integer between 0 and 3.

10. A compound for use according to any one of the preceding claims wherein R represents -(CH₂)₂CH₃.

11. A compound for use according to any one of the preceding claims wherein the compound is an alkyl ester of a monohydroxybenzoic acid, for example an alkyl ester of 2-hydroxybenzoic acid (salicylic acid), 3-hydroxybenzoic acid or 4-hydroxybenzoic acid.

12. A compound for use according to any one of Claims 1 to 10 wherein the compound is an alkyl ester of a dihydroxybenzoic acid, for example an alkyl ester of 2,3-hydroxybenzoic acid, 2,4-hydroxybenzoic acid, 2,5-hydroxybenzoic acid or 2,6-hydroxybenzoic acid.

13. A compound for use according to any one of Claims 1 to 10 wherein the compound is an alkyl ester of a trihydroxybenzoic acid, for example an alkyl ester of 3,4,5-trihydroxybenzoic acid (gallic acid) or 2,4,6-trihydroxybenzoic acid (phloro-glucinol carboxylic acid).

14. A compound for use according to any one of Claims 1 to 10 wherein the compound is an alkyl ester of a tetrahydroxybenzoic acid or of a pentahydroxybenzoic acid.

15. A compound for use according to any one of the preceding claims wherein the compound is provided in a form suitable for delivery to the mucosa of the mouth and/or pharynx.

16. A compound for use according to any one of the preceding claims wherein the compound is for use in combination with a polypeptide having protease activity.

17. A compound for use according to any one of the preceding claims wherein the compound is for use in combination with one or more additional active agents.

18. A compound for use according to Claim 17 wherein the additional active agents are selected from the group consisting of antimicrobial agents (including antibiotics, antiviral agents and anti-fungal agents), anti-inflammatory agents (including steroids and non-steroidal anti-inflammatory agents) and antiseptic agents.

19. A compound for use according to Claim 18 wherein the one or more antimicrobial agents are antibiotics selected from the group consisting of penicillins, cephalosporins, fluoroquinolones, aminoglycosides, monobactams, carbapenems and macrolides.

## Patentansprüche

1. Verbindung der Formel I zur Verwendung bei der Behandlung oder Vorbeugung einer Rhinovirus-Infektion bei einem Säuger 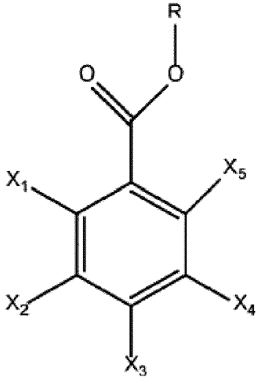 wobei:
R eine C₁₋₁₀-Alkylgruppe darstellt;
X₁, X₂, X₃, X₄ und X₅ unabhängig -H oder -OH darstellen; und
wobei X₁, X₂, X₃, X₄ und/oder X₅ -OH ist.

2. Verbindung zur Verwendung nach Anspruch 1, wobei die Infektion in den oberen Atemwegen auftritt.

3. Verbindung zur Verwendung nach Anspruch 1 oder 2, wobei die Infektion eine Erkältung ist.

4. Verbindung zur Verwendung nach einem der vorhergehenden Ansprüche, wobei das Rhinovirus ein humanes Rhinovirus ist.

5. Verbindung zur Verwendung nach einem der vorhergehenden Ansprüche, wobei das
Rhinovirus Typ A ist und aus der Gruppe ausgewählt ist, die aus humanen Rhinovirus-Serotypen HRV-A1, HRV-A2, HRV-A7, HRV-A8, HRV-A9, HRV-A10, HRV-A11, HRV-A12, HRV-A13, HRV-A15, HRV-A16, HRV-A18, HRV-A19, HRV-A20, HRV-A21, HRV-A22, HRV-A23, HRV-A24, HRV-A25, HRV-A28, HRV-A29, HRV-A30, HRV-A31, HRV-A32, HRV-A33, HRV-A34, HRV-A36, HRV-A38, HRV-A39, HRV-A40, HRV-A41, HRV-A43, HRV-A44, HRV-A45, HRV-A46, HRV-A47, HRV-A49, HRV-A50, HRV-A51, HRV-A53, HRV-A54, HRV-A55, HRV-A56, HRV-A57, HRV-A58, HRV-A59, HRV-A60, HRV-A61, HRV-A62, HRV-A63, HRV-A64, HRV-A65, HRV-A66, HRV-A67, HRV-A68, HRV-A71, HRV-A73, HRV-A74, HRV-A75, HRV-A76, HRV-A77, HRV-A78, HRV-A80, HRV-A81, HRV-A82, HRV-A85, HRV-A88, HRV-A89, HRV-A90, HRV-A94, HRV-A95, HRV-A96, HRV-A98, HRV-A100, HRV-A101, HRV-A102 und HRV-A103 besteht oder ein humanes Rhinovirus ist.
oder wobei das Rhinovirus Typ B ist und aus der Gruppe ausgewählt ist, die aus humanen Rhinovirus-Serotypen HRV-B3, HRV-B4, HRV-B5, HRV-B6, HRV-B14, HRV-B17, HRV-B26, HRV-B27, HRV-B35, HRV-B37, HRV-B42, HRV-B48, HRV-B52, HRV-B69, HRV-B70, HRV-B72, HRV-B79, HRV-B83, HRV-B84, HRV-B86, HRV-B91, HRV-B92, HRV-B93, HRV-B97 und HRV-B99 besteht oder ein humanes Rhinovirus ist.
oder wobei das Rhinovirus Typ C ist und aus der Gruppe ausgewählt ist, die aus humanen Rhinovirus-Serotypen HRV-C1, HRV-C2, HRV-C3, HRV-C4, HRV-C5, HRV-C6, HRV-C7, HRV-C8, HRV-C9, HRV-C10, HRV-C11, HRV-C12, HRV-C13, HRV-C14, HRV-C15, HRV-C16, HRV-C17, HRV-C18, HRV-C19, HRV-C20, HRV-C21, HRV-C22, HRV-C23, HRV-C24, HRV-C25, HRV-C26, HRV-C27, HRV-C28, HRV-C29, HRV-C30, HRV-C31, HRV-C32, HRV-C33, HRV-C34, HRV-C35, HRV-C36, HRV-C37, HRV-C38, HRV-C39, HRV-C40, HRV-C41, HRV-C42, HRV-C43, HRV-C44, HRV-C45, HRV-C46, HRV-C47, HRV-C48, HRV-C49, HRV-C50 und HRV-C51 besteht.

6. Verbindung zur Verwendung nach einem der vorhergehenden Ansprüche, wobei R eine unsubstituierte Alkylgruppe darstellt.

7. Verbindung zur Verwendung nach einem der vorhergehenden Ansprüche, wobei R eine C₁₋₈-Alkylgruppe darstellt, beispielsweise eine C₁₋₆-Alkylgruppe, eine C₁₋₅-Alkylgruppe, eine C₁₋₄-Alkylgruppe, eine C₁₋₃-Alkylgruppe, eine C₁₋₂-Alkylgruppe oder eine C₁-Alkylgruppe.

8. Verbindung zur Verwendung nach einem der vorhergehenden Ansprüche, wobei R eine Methylgruppe, eine Ethylgruppe, eine Propylgruppe oder eine Butylgruppe darstellt.

9. Verbindung zur Verwendung nach einem der vorhergehenden Ansprüche, wobei R - (CH₂)ₙCH₃ darstellt, wobei "n" eine ganze Zahl zwischen 0 und 3 ist.

10. Verbindung zur Verwendung nach einem der vorhergehenden Ansprüche, wobei R - (CH₂)₂CH₃ darstellt.

11. Verbindung zur Verwendung nach einem der vorhergehenden Ansprüche, wobei die Verbindung ein Alkylester einer Monohydroxybenzoesäure ist, beispielsweise ein Alkylester von 2-Hydroxybenzoesäure (Salicylsäure), 3-Hydroxybenzoesäure oder 4-Hydroxybenzoesäure.

12. Verbindung zur Verwendung nach einem der Ansprüche 1 bis 10, wobei die Verbindung ein Alkylester einer Dihydroxybenzoesäure ist, beispielsweise ein Alkylester von 2,3-Hydroxybenzoesäure, 2,4-Hydroxybenzoesäure, 2,5-Hydroxybenzoesäure oder 2,6-Hydroxybenzoesäure.

13. Verbindung zur Verwendung nach einem der Ansprüche 1 bis 10, wobei die Verbindung ein Alkylester einer Trihydroxybenzoesäure ist, beispielsweise ein Alkylester von 3,4,5-Trihydroxybenzoesäure (Gallussäure) oder 2,4,6-Trihydroxybenzoesäure (Phloro-Glucinol-Carbonsäure).

14. Verbindung zur Verwendung nach einem der Ansprüche 1 bis 10, wobei die Verbindung ein Alkylester einer Tetrahydroxybenzoesäure oder einer Pentahydroxybenzoesäure ist.

15. Verbindung zur Verwendung nach einem der vorhergehenden Ansprüche, wobei die Verbindung in einer Form bereitgestellt ist, die für eine Abgabe an die Mund- und/oder Rachenschleimhaut geeignet ist.

16. Verbindung zur Verwendung nach einem der vorhergehenden Ansprüche, wobei die Verbindung zur Verwendung in Kombination mit einem Polypeptid vorgesehen ist, das eine Proteaseaktivität aufweist.

17. Verbindung zur Verwendung nach einem der vorhergehenden Ansprüche, wobei die Verbindung zur Verwendung in Kombination mit einem oder mehreren zusätzlichen Wirkstoffen vorgesehen ist.

18. Verbindung zur Verwendung nach Anspruch 17, wobei die zusätzlichen Wirkstoffe aus der Gruppe ausgewählt sind, die aus antimikrobiellen Mitteln (einschließlich Antibiotika, antiviralen Mitteln und antimykotischen Mitteln), entzündungshemmenden Mitteln (einschließlich Steroiden und nicht-steroidalen entzündungshemmenden Mitteln) und antiseptischen Mitteln besteht.

19. Verbindung zur Verwendung nach Anspruch 18, wobei das eine oder die mehreren antimikrobiellen Mittel Antibiotika sind, die aus der Gruppe ausgewählt sind, die aus Penicillinen, Cephalosporinen, Fluorchinolonen, Aminoglykosiden, Monobactamen, Carbapenemen und Makroliden besteht.

## Revendications

1. Composé de formule I destiné à être utilisé dans le traitement ou la prévention d'une infection à rhinovirus chez un mammifère 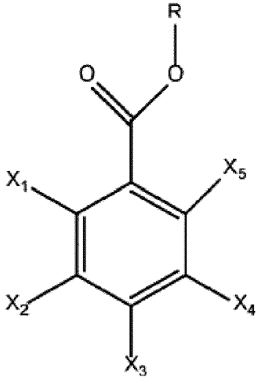
R représentant un groupe alkyle en C₁ à C₁₀ ;
X₁, X₂, X₃, X₄ et X₅ représentant indépendamment -H ou -OH ; et
X₁ et/ou X₂ et/ou X₃ et/ou X₄ et/ou X₅ étant -OH.

2. Composé destiné à être utilisé selon la revendication 1, l'infection se situant dans les voies respiratoires supérieures.

3. Composé destiné à être utilisé selon la revendication 1 ou 2, l'infection étant un rhume simple.

4. Composé destiné à être utilisé selon l'une quelconque des revendications précédentes, le rhinovirus étant un rhinovirus humain.

5. Composé destiné à être utilisé selon l'une quelconque des revendications précédentes, le rhinovirus étant de type A et étant choisi dans le groupe constitué des sérotypes de
rhinovirus humains HRV-A1, HRV-A2, HRV-A7, HRV-A8, HRV-A9, HRV-A10, HRV-A11, HRV-A12, HRV-A13, HRV-A15, HRV-A16, HRV-A18, HRV-A19, HRV-A20, HRV-A21, HRV-A22, HRV-A23, HRV-A24, HRV-A25, HRV-A28, HRV-A29, HRV-A30, HRV-A31, HRV-A32, HRV-A33, HRV-A34, HRV-A36, HRV-A38, HRV-A39, HRV-A40, HRV-A41, HRV-A43, HRV-A44, HRV-A45, HRV-A46, HRV-A47, HRV-A49, HRV-A50, HRV-A51, HRV-A53, HRV-A54, HRV-A55, HRV-A56, HRV-A57, HRV-A58, HRV-A59, HRV-A60, HRV-A61, HRV-A62, HRV-A63, HRV-A64, HRV-A65, HRV-A66, HRV-A67, HRV-A68, HRV-A71, HRV-A73, HRV-A74, HRV-A75, HRV-A76, HRV-A77, HRV-A78, HRV-A80, HRV-A81, HRV-A82, HRV-A85, HRV-A88, HRV-A89, HRV-A90, HRV-A94, HRV-A95, HRV-A96, HRV-A98, HRV-A100, HRV-A101, HRV-A102 et HRV-A103, ou étant un rhinovirus humain.
ou le rhinovirus étant de type B et étant choisi dans le groupe constitué des sérotypes de rhinovirus humains HRV-B3, HRV-B4, HRV-B5, HRV-B6, HRV-B14, HRV-B17, HRV-B26, HRV-B27, HRV-B35, HRV-B37, HRV-B42, HRV-B48, HRV-B52, HRV-B69, HRV-B70, HRV-B72, HRV-B79, HRV-B83, HRV-B84, HRV-B86, HRV-B91, HRV-B92, HRV-B93, HRV-B97 et HRV-B99, ou étant un rhinovirus humain.
ou le rhinovirus étant de type C et étant choisi dans le groupe constitué des sérotypes de rhinovirus humains HRV-C1, HRV-C2, HRV-C3, HRV-C4, HRV-C5, HRV-C6, HRV-C7, HRV-C8, HRV-C9, HRV-C10, HRV-C11, HRV-C12, HRV-C13, HRV-C14, HRV-C15, HRV-C16, HRV-C17, HRV-C18, HRV-C19, HRV-C20, HRV-C21, HRV-C22, HRV-C23, HRV-C24, HRV-C25, HRV-C26, HRV-C27, HRV-C28, HRV-C29, HRV-C30, HRV-C31, HRV-C32, HRV-C33, HRV-C34, HRV-C35, HRV-C36, HRV-C37, HRV-C38, HRV-C39, HRV-C40, HRV-C41, HRV-C42, HRV-C43, HRV-C44, HRV-C45, HRV-C46, HRV-C47, HRV-C48, HRV-C49, HRV-C50 et HRV-C51.

6. Composé destiné à être utilisé selon l'une quelconque des revendications précédentes, R représentant un groupe alkyle non substitué.

7. Composé destiné à être utilisé selon l'une quelconque des revendications précédentes, R représentant un groupe alkyle en C₁ à C₈, par exemple un groupe alkyle en C₁ à C₆, un groupe alkyle en C₁ à C₅, un groupe alkyle en C₁ à C₄, un groupe alkyle en C₁ à C₃, un groupe alkyle en C₁ à C₂ ou un groupe alkyle en C₁.

8. Composé destiné à être utilisé selon l'une quelconque des revendications précédentes, R représentant un groupe méthyle, un groupe éthyle, un groupe propyle ou un groupe butyle.

9. Composé destiné à être utilisé selon l'une quelconque des revendications précédentes, R représentant -(CH₂)ₙCH₃, 'n' étant un nombre entier compris entre 0 et 3.

10. Composé destiné à être utilisé selon l'une quelconque des revendications précédentes, R représentant -(CH₂)₂CH₃.

11. Composé destiné à être utilisé selon l'une quelconque des revendications précédentes, le composé étant un ester alkyle d'un acide monohydroxybenzoïque, par exemple un ester alkyle d'acide 2-hydroxybenzoïque (acide salicylique), d'acide 3-hydroxybenzoïque ou d'acide 4-hydroxybenzoïque.

12. Composé destiné à être utilisé selon l'une quelconque des revendications 1 à 10, le composé étant un ester alkyle d'un acide dihydroxybenzoïque, par exemple un ester alkyle d'acide 2,3-hydroxybenzoïque, d'acide 2,4-hydroxybenzoïque, d'acide 2,5-hydroxybenzoïque ou d'acide 2,6-hydroxybenzoïque.

13. Composé destiné à être utilisé selon l'une quelconque des revendications 1 à 10, le composé étant un ester alkyle d'un acide trihydroxybenzoïque, par exemple un ester alkyle d'acide 3,4,5-trihydroxybenzoïque (acide gallique) ou d'acide 2,4,6-trihydroxybenzoïque (acide phloroglucinol carboxylique).

14. Composé destiné à être utilisé selon l'une quelconque des revendications 1 à 10, le composé étant un ester alkyle d'un acide tétrahydroxybenzoïque ou d'un acide pentahydroxybenzoïque.

15. Composé destiné à être utilisé selon l'une quelconque des revendications précédentes, le composé étant fourni sous une forme appropriée pour l'administration à la muqueuse de la bouche et/ou du pharynx.

16. Composé destiné à être utilisé selon l'une quelconque des revendications précédentes, le composé étant destiné à être utilisé en combinaison avec un polypeptide ayant une activité protéasique.

17. Composé destiné à être utilisé selon l'une quelconque des revendications précédentes, le composé étant destiné à être utilisé en combinaison avec un ou plusieurs agents actifs supplémentaires.

18. Composé destiné à être utilisé selon la revendication 17, les agents actifs supplémentaires étant choisis dans le groupe constitué d'agents antimicrobiens (y compris les antibiotiques, les agents antiviraux et les antifongiques), d'anti-inflammatoires (y compris les stéroïdes et les anti-inflammatoires non stéroïdiens) et d'agents antiseptiques.

19. Composé destiné à être utilisé selon la revendication 18, le ou les agents antimicrobiens étant des antibiotiques choisis dans le groupe constitué de pénicillines, de céphalosporines, de fluoroquinolones, d'aminoglucosides, de monobactames, de carbapénèmes et de macrolides.
